(19) Europäisches Patentamt
European Patent Office
Office européen des brevets

(11) **EP 3 644 199 A1**

(12) **EUROPEAN PATENT APPLICATION**
published in accordance with Art. 153(4) EPC

(43) Date of publication:
**29.04.2020 Bulletin 2020/18**

(21) Application number: **18752079.6**

(22) Date of filing: **25.01.2018**

(51) Int Cl.:
***G06F 19/00*** ***(2018.01)***

(86) International application number:
**PCT/CN2018/074158**

(87) International publication number:
**WO 2018/233289 (27.12.2018 Gazette 2018/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(30) Priority: **21.06.2017 CN 201710476500**

(71) Applicant: **BOE Technology Group Co., Ltd.
Beijing 100015 (CN)**

(72) Inventors:
• **LIANG, Xuan
  Beijing 100176 (CN)**
• **ZHANG, Chao
  Beijing 100176 (CN)**

(74) Representative: **Jacobacci Coralis Harle
32, rue de l'Arcade
75008 Paris (FR)**

(54) **MEDICAL DATA MATCHING DEVICE AND METHOD**

(57) The present disclosure provides a device and a method for matching medical data. The device includes: a computing device configured to compute an overall similarity between a current object and each object in a set of objects, wherein the overall similarity is determined by one or more of an original region similarity, a living region similarity, a relevant physiological parameter similarity and a critical time similarity; and a selector configured to select, from the set of objects, an object with the overall similarity greater than a first threshold or a predetermined number of or a predetermined proportion of objects with the overall similarities being ranked on the top, to form a reference object subset for the current object.

FIG. 1

**Description**

TECHNICAL FIELD

Embodiments of the present disclosure relate to the field of medical data, and more particularly, to a device and a method for matching medical data.

BACKGROUND

Most of medical data at the present stage are from patient registration, outpatient service, and hospital charges. Only a small amount of information is from the systems of patients' case information. This information is about patients' illness state, which does not take into consideration various factors' effects on the patients' illness state. In the meanwhile, it is difficult to find out other patient populations having similar medical histories with respect to the current patient' medical history, from a large medical database, by retrieving data to make an analysis and prediction of the current patient' illness state.

Thus, a solution for matching medical data is required in this art.

SUMMARY

An aspect of the present disclosure provides a device for matching medical data. The device includes: a computing device configured to compute an overall similarity between a current object and each object in a set of objects, wherein the overall similarity is determined by one or more of an original region similarity, a living region similarity, a relevant physiological parameter similarity and a critical time similarity between the current object and each object in the set of objects; and a selector configured to select, from the set of objects, an object with the overall similarity greater than a first threshold or a predetermined number of or a predetermined proportion of objects with the overall similarities being ranked on the top, to form a reference object subset for the current object.

In at least one embodiment of the present disclosure, the overall similarity is computed as a weighted sum of one or more of the original region similarity, the living region similarity, the relevant physiological parameter similarity and the critical time similarity.

In at least one embodiment of the present disclosure, the original region similarity represents proximity between geographical locations of ancestral homes of the objects.

In at least one embodiment of the present disclosure, the original region similarity is computed based on following formula:

$$OH_{i,j} = \left| \frac{L - OD_{i,j}}{L + OD_{i,j}} \right|,$$

wherein $OH_{i,j}$ represents the original region similarity between the objects i and j, $OD_{i,j}$ represents a distance between the ancestral homes of the objects i and j, and L represents a half of the earth's equatorial circumference.

In at least one embodiment of the present disclosure, the living region similarity represents proximity between geographical locations of living regions of the objects.

In at least one embodiment of the present disclosure, the living region similarity is computed based on following formula:

$$LA_{i,j} = \left| \frac{L - LD_{i,j}}{L + LD_{i,j}} \right|,$$

wherein $LA_{i,j}$ represents the living region similarity between the objects i and j, $LD_{i,j}$ represents a distance between the living regions of the objects i and j, and L represents a half of the earth's equatorial circumference.

In at least one embodiment of the present disclosure, the relevant physiological parameter similarity represents a similarity degree between relevant physiological parameters of the objects.

In at least one embodiment of the present disclosure, the relevant physiological parameter similarity is computed based on following formula:

$$ST_{i,j} = \frac{2S_i \cdot S_j^T}{(S_i \cdot C^T + S_j \cdot C^T)},$$

wherein $ST_{i,j}$ represents the relevant physiological parameter similarity between the objects i and j, $S_i$ represents a relevant physiological parameter vector of the object i, $S_j^T$ represents a transposition of the relevant physiological parameter vector of the object j, $C^T$ represents a transposition of a vector with all elements being 1, and wherein each element of the relevant physiological parameter vector corresponds to one symptom, and if an object has a certain symptom, the element corresponding to the symptom of the relevant physiological parameter vector of the object is set to 1, otherwise the element is set to 0.

In at least one embodiment of the present disclosure, the critical time similarity represents a similarity degree between the durations for disease symptoms of the objects.

In at least one embodiment of the present disclosure, the critical time similarity is computed based on following formula:

$$TF_{i,j} = \sum_{k-1}^{n}(1 - \frac{|T_{ik} - T_{jk}|}{T_{ik} + T_{jk}})/n,$$

wherein $TF_{i,j}$ represents the critical time similarity between the objects i and j, $T_{ik}$ represents a duration for the $k^{th}$ symptom of the object i, $T_{jk}$ represents a duration for the $k^{th}$ symptom of the object j, and n represents a total number of symptoms.

Another aspect of the present disclosure provides a method for matching medical data. The method includes: computing an overall similarity between a current object and each object in a set of objects, wherein the overall similarity is determined by one or more of an original region similarity, a living region similarity, a relevant physiological parameter similarity, and a critical time similarity between the current object and each object in the set of objects; and selecting, from the set of objects, an object with the overall similarity greater than a first threshold or a predetermined number of or a predetermined proportion of objects with the overall similarities being ranked on the top, to form a reference object subset for the current object.

In at least one embodiment of the present disclosure, the overall similarity is computed as a weighted sum of one or more of the original region similarity, the living region similarity, the relevant physiological parameter similarity and the critical time similarity.

In at least one embodiment of the present disclosure, the original region similarity represents proximity between geographical locations of ancestral homes of the objects.

In at least one embodiment of the present disclosure, the original region similarity is computed based on following formula:

$$OH_{i,j} = \left| \frac{L - OD_{i,j}}{L + OD_{i,j}} \right|,$$

wherein $OH_{i,j}$ represents the original region similarity between the objects i and j, $OD_{i,j}$ represents a distance between the ancestral homes of the objects i and j, and L represents a half of the earth's equatorial circumference.

In at least one embodiment of the present disclosure, the living region similarity represents proximity between geographical locations of living regions of the objects.

In at least one embodiment of the present disclosure, the living region similarity is computed based on following formula:

$$LA_{i,j} = \left| \frac{L - LD_{i,j}}{L + LD_{i,j}} \right|,$$

wherein $LA_{i,j}$ represents the living region similarity between the objects i and j, $LD_{i,j}$ represents a distance between the living regions of the objects i and j, and L represents a half of the earth's equatorial circumference.

In at least one embodiment of the present disclosure, the relevant physiological parameter similarity represents a similarity degree between disease symptoms of the objects.

In at least one embodiment of the present disclosure, the relevant physiological parameter similarity is computed based on following formula:

$$ST_{i,j} = \frac{2S_i \cdot S_j^T}{(S_i \cdot C^T + S_j \cdot C^T)},$$

wherein $ST_{i,j}$ represents the relevant physiological parameter similarity between the objects i and j, $S_i$ represents a relevant physiological parameter vector of the object i, $S_j^T$ represents a transposition of the relevant physiological parameter vector of the object j, $C^T$ represents a transposition of a vector with all elements being 1, and wherein each element of the relevant physiological parameter vector corresponds to one symptom, and if an object has a certain symptom, the element corresponding to the symptom of the relevant physiological parameter vector of the object is set to 1, otherwise the element is set to 0.

In at least one embodiment of the present disclosure, the critical time similarity represents a similarity degree between the durations for disease symptoms of the objects.

In at least one embodiment of the present disclosure, the critical time similarity is computed based on following formula:

$$TF_{i,j} = \sum_{k=1}^{n}(1 - \frac{|T_{ik} - T_{jk}|}{T_{ik} + T_{jk}})/n,$$

wherein $TF_{i,j}$ represents the critical time similarity between the objects i and j, $T_{ik}$ represents a duration for the $k^{th}$ symptom of the object i, $T_{jk}$ represents a duration for the $k^{th}$ symptom of the object j, and n represents a total number of symptoms.

Still another aspect of the present disclosure further provides a computer system, which includes a processor and a memory connected to the processor. The memory stores program instructions. The processor is configured to perform the method for matching medical data according to any one embodiment of the present disclosure by loading and executing the program instructions in the memory.

Still another aspect of the present disclosure further provides a storage medium storing program instructions. The program instructions can be loaded and executed by a processor to perform the method for matching medical data according to any one embodiment of the present disclosure.

BRIEF DESCRIPTION OF DRAWINGS

FIG. 1 illustrates a device for matching medical data according to an embodiment of the present disclosure; and

FIG. 2 illustrates a method for matching medical data according to an embodiment of the present disclosure.

DETAILED DESCRIPTION

In order that those skilled in the art could better understand the technical solution of the present disclosure, in the following the method and the device for matching medical data provided by embodiments of the present disclosure are further described in detail with reference to the accompanying drawings and embodiments. Apparently, the described embodiments are some but not all of the embodiments of the present disclosure. All other embodiments obtained by those of ordinary skill in the art based on the described embodiments of the present disclosure without creative efforts shall fall within the protection scope of the present disclosure.

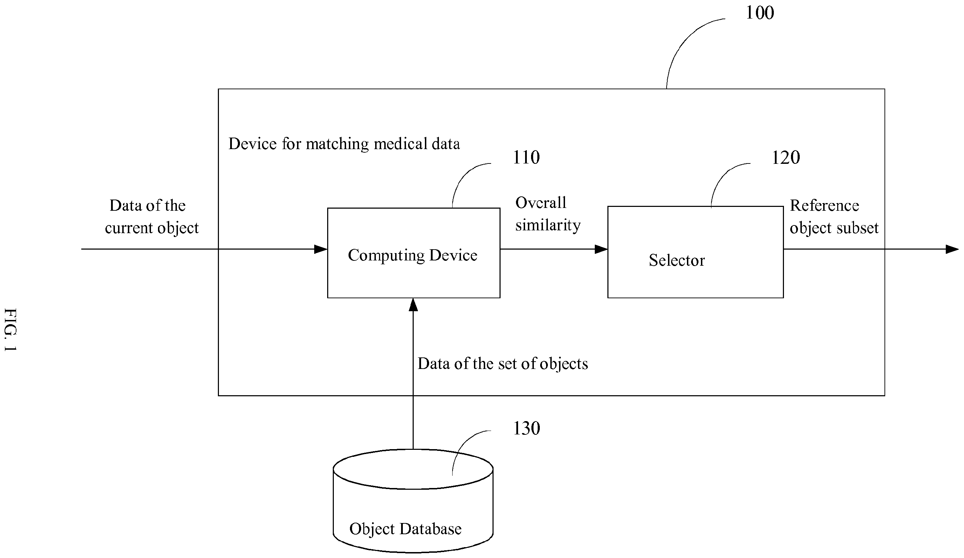

Referring to FIG. 1, FIG. 1 illustrates a device 100 for matching medical data according to an embodiment of the present disclosure. As shown in FIG. 1, the device 100 for matching medical data includes: a computing device 110, configured to compute an overall similarity between a current object and each object in a set of objects, wherein the overall similarity is determined by one or more of an original region similarity, a living region similarity, a relevant physiological parameter similarity and a critical time similarity between the current object and each object in the set of objects; and a selector 120, configured to select, from the set of objects, an object with the overall similarity greater than a first threshold or a predetermined number of or a predetermined proportion of objects with the overall similarities being ranked on the top, to form a reference object subset for the current object.

That is, the computing device 110 may first compute one or more of the original region similarity, the living region similarity, the relevant physiological parameter similarity and the critical time similarity between the current object and each object in the set of objects, and then compute the overall similarity between the current object and each object in the set of objects based on the one or more of the original region similarity, the living region similarity, the relevant physiological parameter similarity and the critical time similarity, such that the selector 120 selects objects with the greater overall similarity with respect to the current object, to form the reference object subset for the current object.

Alternatively, the computing device 110 also may first compute one of the original region similarity, the living region similarity, the relevant physiological parameter similarity and the critical time similarity between the current object and each object in the set of objects, and the selector 120 selects, from the set of objects, an object having the greater value for the one of the original region similarity, the living region similarity, the relevant physiological parameter similarity and the critical time similarity with respect to the current object to form an intermediate subset of objects. Next, the computing device 110 may further compute another one of the original region similarity, the living region similarity, the relevant physiological parameter similarity and the critical time similarity between the current object and each object in the intermediate subset of objects, and the selector 120 selects, from the intermediate subset of objects, another object having the greater value for the another one of the original region similarity, the living region similarity, the relevant physiological parameter similarity and the critical time similarity with respect to the current object to form a further intermediate subset of patients (or a subset of reference patients) and the process is going on in the same way until the reference object subset is formed.

The computing device 110 may receive one or more of original region data, living region data, relevant physiological parameter data and critical time data of the current object, and obtain the corresponding one or more of original region data, living region data, relevant physiological parameter data and critical time data of each object in the set of objects, then compare the one or more of the original region data, the living region data, the relevant physiological parameter data and the critical time data of the current object with the corresponding one or more of the original region data, the living region data, the relevant physiological parameter data and the critical time data of each object in the set of objects to compute one or more of the original region similarity, the living region similarity, the relevant physiological parameter similarity and the critical time similarity between the current object and each object in the set of objects.

The set of objects may include a large number of objects, and the original region data, the living region data, the relevant physiological parameter data and the critical time data of each object in the set of objects may be stored in an object database 130.

In some embodiments of the present disclosure, the object may be a patient, the original region may be an ancestral home, the living region may be a resident region, the relevant physiological parameter may be a relevant symptom, and the critical time may be duration for the relevant symptom.

It is recognized by the inventor that many diseases are characterized by regional heredity and environmental influence. Influences of the patients' ancestral homes and living regions on the patients are very important. In the meanwhile, in consideration of factors such as the patients' existing symptoms and disease durations, similar patients having matched characteristics with respect to the current patients are found out from a patient database, which is of important reference for prediction and/or diagnosis of the current patient's state of illness, and is conducive to health research or statistics for specific populations.

Factors of ancestral homes may represent influences of relevant region diseases in living regions of the patients' families on the patients. People having the same or similar ancestral homes are apt to having a possibility of suffering from the same region and hereditary diseases. The ancestral homes may be living regions of the patients' parents, grandparents, and even great-great-grandparents.

In some embodiments, the original region similarity represents proximity between geographical locations of ancestral homes of the objects.

In some further embodiments, the original region similarity may be computed based on following formula:

$$OH_{i,j} = \left| \frac{L - OD_{i,j}}{L + OD_{i,j}} \right|, \tag{1}$$

wherein $OH_{i,j}$ represents the original region similarity between the objects i and j, $OD_{i,j}$ represents a distance between the ancestral homes of the objects i and j, and L represents a half of the earth's equatorial circumference.

Based on the above Formula (1), the smaller the distance between ancestral home regions of two objects is, the greater the original region similarity is. The $OD_{i,j}$ between two objects having the same ancestral home is 0, and thus the original region similarity $OH_{i,j}$ thereof is the maximum value 1. The $OD_{i,j}$ between two objects having the ancestral home distance being a half of the earth's equatorial circumference is the maximum value L, and thus the original region similarity $OH_{i,j}$ thereof is the minimum value 0.

The distance $OD_{i,j}$ between ancestral homes of objects i and j may be computed based on the geographic coordinates of the ancestral homes of the objects i and j. Supposing the geographic coordinates (supposing a rectangular coordinate system is used) of the ancestral homes of the objects i and j are $(x_i, y_i)$ and $(x_j, y_j)$ respectively, the distance $OD_{i,j}$ between the ancestral homes of the objects i and j may be computed based on following formula:

$$OD_{i,j} = \sqrt{(x_i - x_j)^2 + (y_i - y_j)^2}, \qquad (2)$$

Of course, the above Formulas (1) and (2) are merely exemplary, but are not intended for limiting the present disclosure. In other embodiments of the present disclosure, the original region similarity between the objects may be computed based on other formulas.

Living region factors mainly represent effects of environmental factors and human factors or the like (including air quality, quality of domestic water, personnel quality, a fact whether regional infectious diseases outbroke) of the living region of the object on the object. Objects having the same or similar living regions are apt to having a possibility of suffering from the same region disease(s).

In some embodiments, the living region similarity represents proximity between geographical locations of living regions of the objects.

In some further embodiments, the living region similarity is computed based on following formula:

$$LA_{i,j} = \left| \frac{L - LD_{i,j}}{L + LD_{i,j}} \right|, \qquad (3)$$

wherein $LA_{i,j}$ represents the living region similarity between the objects i and j, $LD_{i,j}$ represents a distance between the living regions of the objects i and j, and L represents a half of the earth's equatorial circumference.

Based on the above Formula (3), the smaller the distance between the living regions of two objects is, the greater the living region similarity is. The $LD_{i,j}$ between two objects having the same living region is 0, and thus the living region similarity $LA_{i,j}$ thereof is the maximum value 1. The $LD_{i,j}$ between two objects having the living region distance being a half of the earth's equatorial circumference is the maximum value L, the living region similarity $LA_{i,j}$ thereof is the minimum value 0.

Of course, the above Formula (3) is merely exemplary, but is not intended for limiting the present disclosure. In other embodiments of the present disclosure, the living region similarity between the objects may be computed based on other formulas.

Relevant symptom factors mainly reflect similarity comparison of disease symptoms of the objects and medical history of other objects. Objects having the same or similar disease symptoms are apt to having the same or similar diseases.

In some embodiments, the relevant physiological parameter similarity represents a similarity degree between disease symptoms of the objects.

In some further embodiments, the relevant physiological parameter similarity includes an external symptom similarity and an internal symptom similarity, which respectively represent a similarity degree between external disease symptoms of the objects and a similarity degree between internal disease symptoms of the objects. The internal disease symptoms may include, for example, bronchial inflammation, abnormalities of various internal organs such as heart, liver, spleen, lung, kidney, stomach, large intestine, small intestine, triple burner, bladder and gallbladder, or abnormalities of physiological functions. The external disease symptoms may include abnormalities of external functions or representations, for example, cold, fever, rhinorrhea, allergy, and so on.

In some further embodiments, the relevant physiological parameter similarity is computed based on following formula:

$$ST_{i,j} = \frac{2S_i \cdot S_j^T}{(S_i \cdot C^T + S_j \cdot C^T)}, \qquad (4)$$

wherein $ST_{i,j}$ represents the relevant physiological parameter similarity between the objects i and j, $S_i$ represents a relevant symptom vector of the object i, $S_j^T$ represents a transposition of the relevant symptom vector of the object j, $C^T$ represents a transposition of a vector with all elements being 1, and wherein each element of the relevant symptom vector corresponds to one symptom. If an object has a certain symptom, the element corresponding to the symptom of the relevant symptom vector of the object is set to 1, otherwise the element is set to 0.

Based on the above Formula (4), the more the same symptoms having by the two objects, the greater the relevant physiological parameter similarity thereof is. The relevant physiological parameter similarity of two objects having all the same symptoms has the maximum value 1. The relevant physiological parameter similarity of two objects having no common symptom has the minimum value 0.

Of course, the above Formula (4) is merely exemplary, but is not intended for limiting the present disclosure.

In other embodiments of the present disclosure, the relevant physiological parameter similarity between the two objects may be computed based on other formulas.

In some other embodiments, the relevant physiological parameter similarity is computed in consideration of a similarity between types and severity degrees of one or more symptoms of different objects. That is, when the relevant physiological parameter similarity between the two objects is computed, the severity degree or level of each symptom may also be considered. The closer the severity degree or level of the symptoms is, the greater the relevant physiological parameter similarity of the objects is.

In some embodiments, the critical time similarity represents a similarity degree between the durations for disease symptoms of the objects.

In some further embodiments, the critical time similarity is computed in consideration of a proximity between durations for one or more similar symptoms of different objects.

More specifically, the critical time similarity is computed based on following formula:

$$TF_{i,j} = \sum_{k=1}^{n}\left(1 - \frac{|T_{ik} - T_{jk}|}{T_{ik} + T_{jk}}\right)/n. \qquad (5)$$

wherein $TF_{i,j}$ represents the critical time similarity between the objects i and j, $T_{ik}$ represents a duration for the $k^{th}$ symptom of the object i, $T_{jk}$ represents a duration for the $k^{th}$ symptom of the object j, and n represents a total number of symptoms.

Based on the above Formula (5), the closer the durations for the same symptoms of two objects is, the greater the critical time similarity of the two objects is. If all the symptoms of the two objects are the same and the durations are equal, the critical time similarity of the two objects has the maximum value 1. If the durations of each corresponding symptom of either object of the two objects are close to 0, the critical time similarity of the two objects is close to the minimum value 0.

Of course, the above Formula (5) is merely exemplary, but is not intended for limiting the present disclosure. In other embodiments of the present disclosure, the critical time similarity between the two objects may be computed based on other formulas.

In some embodiments, the overall similarity is computed as a weighted sum of one or more of the original region similarity, the living region similarity, the relevant physiological parameter similarity and the critical time similarity, and is formulated as below:

$$GS_{i,j} = \omega_1 * OH_{i,j} + \omega_2 * LA_{i,j} + \omega_3 * ST_{ij} + \omega_4 * TF_{ij}, \quad (6)$$

wherein $GS_{i,j}$ represents the overall similarity between the objects i and j, $\omega_1$, $\omega_2$, $\omega_3$ and $\omega_4$ respectively represent weight of the original region similarity, weight of the living region similarity, weight of relevant physiological parameter similarity, and weight of critical time similarity. Alternatively, $\omega_1 + \omega_2 + \omega_3 + \omega_4 = 1$. A numerical value of each weight may be predetermined in accordance with specific conditions. For example, each weight may be selected as 0.25. Alternatively, different weights may be selected based on the extent of the effect of each factor. For example, if it is believed that the ancestral home has a greater effect on the object's illness state, the weight of the original region similarity may be increase, for example, the $\omega_1$ is selected as 0.5, and weights of other factors are accordingly reduced. For another example, if it is believed that the object's illness state is not affected by the living region at all, the weight $\omega_2$ of the living region similarity may be selected as 0, and weights of other factors are accordingly increased.

The selector 120 may select, from the set of objects, an object having an overall similarity with respect to the current object greater than the overall similarities of the other objects in the set of objects with respect to the current object. For example, the selector 120 may select, from the set of objects, a predetermined number of or a predetermined proportion of objects, wherein the overall similarity between each of the predetermined number or the predetermined proportion of objects and the current object is greater than the overall similarities between the other objects in the set of objects and the current object. For another example, the selector 120 may select, from the set of objects, an object with the greater overall similarity based on distribution characteristics of the overall similarities between the objects in the set of objects and the current object, to form the reference object subset.

The reference object subset obtained in the present disclosure may be provided or presented to relevant personnel for use. For example, the reference object subset is provided or presented to a doctor for reference in a diagnostic or therapeutic process, or the reference object subset is provided or presented to other health researchers for the purpose of health research or statistics, or the reference object subset also may be provided to other systems or devices for other purposes or may be further processed to obtain other useful data.

The device for matching medical data according to the embodiment of the present disclosure has been described above with reference to the accompanying drawings, and it is to be noted that the above description is by way of example only and is not intended to be limiting of the present disclosure. In other embodiments of the present disclosure, the device may have more, fewer or different modules, and the relationship of connection, inclusion, and functionality etc. among the modules may be different from what has been described and illustrated. For example, the object database 130 may also be included in the device. For another example, a plurality of functions generally executed by one module also may be executed by a plurality of individual modules; a plurality of module executing different functions may be combined into one larger module to execute these functions; and functions executed by one module also may be executed by another module, and so on. All these variations fall within the spirit and the scope of the present disclosure.

The device for matching medical data according to the embodiment of the present disclosure may be implemented by hardware, software, firmware or arbitrary combination thereof. The device for matching medical data according to the embodiment of the present disclosure may be implemented in a computer system in a centralized manner or may be implemented in a distributed manner, in which different components are distributed in a plurality of interconnected computer systems. A typical combination of hardware and software may be a general-purpose computer system with computer programs. A program code module in the computer programs corresponds to each module in the device for matching medical data according to the embodiment of the present disclosure. When the computer programs are loaded and executed, the computer system is controlled to execute operations and functions of each module in the device for matching medical data according to the embodiment of the present disclosure.

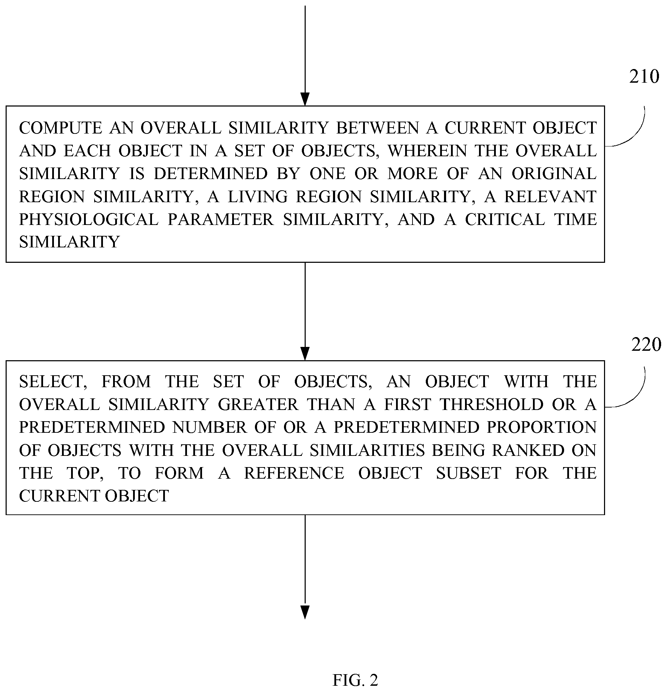

Referring to FIG. 2, another aspect of the present disclosure further provides a method for matching medical data according to an embodiment of the present disclosure. The method for matching medical data may be performed by the device for matching medical data according to the embodiment of the present disclosure, or also may be performed by other devices. Each step of the method for matching medical data may correspond to the operations of each module in the device for matching medical data according to the embodiment of the present disclosure. For the sake of brevity, some details repeated with respect to the above description are omitted in the following description. Therefore, more details on the method for matching medical data according to the embodiment of the present disclosure can be obtained with reference to the above description.

As shown in FIG. 2, the method for matching medical data includes following steps.

In Step 210, an overall similarity between a current object and each object in a set of objects is computed, wherein the overall similarity is determined by one or more of an original region similarity, a living region similarity, a relevant physiological parameter similarity, and a critical time similarity.

In Step 220, an object with the overall similarity greater than a first threshold or a predetermined number of or a predetermined proportion of objects with the overall similarities being ranked on the top are selected from the set of objects to form a reference object subset for the current object.

In some embodiments, the overall similarity is computed as a weighted sum of one or more of the original region similarity, the living region similarity, the relevant physiological parameter similarity and the critical time similarity.

In some embodiments, the original region similarity represents proximity between geographical locations of ancestral homes of the objects.

In some further embodiments, the original region similarity may be computed based on following formula:

$$OH_{i,j} = \left| \frac{L - OD_{i,j}}{L + OD_{i,j}} \right|, \tag{1}$$

wherein $OH_{i,j}$ represents the original region similarity between the objects i and j, $OD_{i,j}$ represents a distance between the ancestral homes of the objects i and j, and L represents a half of the earth's equatorial circumference.

In some embodiments, the living region similarity represents proximity between geographical locations of living regions of the objects.

In some further embodiments, the living region similarity is computed based on following formula:

$$LA_{i,j} = \left| \frac{L - LD_{i,j}}{L + LD_{i,j}} \right|, \tag{3}$$

wherein $LA_{i,j}$ represents the living region similarity between the objects i and j, $LD_{i,j}$ represents a distance between the living regions of the objects i and j, and L represents a half of the earth's equatorial circumference.

In some embodiments, the relevant physiological parameter similarity represents a similarity degree between disease symptoms of the objects.

In some further embodiments, the relevant physiological parameter similarity is computed based on following formula:

$$ST_{i,j} = \frac{2S_i \cdot S_j^T}{(S_i \cdot C^T + S_j \cdot C^T)},\qquad (4)$$

wherein $ST_{i,j}$ represents the relevant physiological parameter similarity between the objects i and j, $S_i$ represents a relevant symptom vector of the object i, $S_j^T$ represents a transposition of the relevant symptom vector of the object j, $C^T$ represents a transposition of a vector with all elements being 1, and wherein each element of the relevant symptom vector corresponds to one symptom, and if an object has a certain symptom, the element corresponding to the symptom in the relevant symptom vector of the object is set to 1, otherwise the element is set to 0.

In some embodiments, the critical time similarity represents a similarity degree between the durations for disease symptoms of the objects.

In some further embodiments, the critical time similarity is computed based on following formula:

$$TF_{i,j} = \sum_{k=1}^{n}\left(1 - \frac{|T_{ik} - T_{jk}|}{T_{ik} + T_{jk}}\right)/n,\qquad (5)$$

wherein $TF_{i,j}$ represents the critical time similarity between the objects i and j, $T_{ik}$ represents a duration for the $k^{th}$ symptom of the object i, $T_{jk}$ represents a duration for the $k^{th}$ symptom of the object j, and n represents a total number of symptoms.

The method for matching medical data according to the embodiments of the present disclosure has been described above with reference to the accompanying drawings, and it is to be noted that the above description is by way of example only and is not intended to be limit of the present disclosure. In other embodiments of the present disclosure, the method may have more, fewer or different steps, and the relationship of sequences, inclusion, and functionality etc. among the steps may be different from what has been described and illustrated. For example, one step may generally be divided into a plurality of individual steps; a plurality of steps may be combined into an individual step; and executions of some steps may have no fixed sequence, etc. All these variations fall within the spirit and the scope of the present disclosure.

The method for matching medical data according to the embodiments of the present disclosure may be implemented by hardware, software, firmware or arbitrary combination thereof. The method for matching medical data according to the embodiment of the present disclosure may be implemented in a computer system in a centralized manner or may be implemented in a distributed manner, in which different components are distributed in a plurality of interconnected computer systems. A typical combination of hardware and software may be a general-purpose computer system with computer programs. When the computer programs are loaded and executed, the computer system is controlled to perform each step in the method for matching medical data according to the embodiments of the present disclosure.

Still another aspect of the present disclosure further provides a computer system, which includes a processor and a memory connected to the processor. The memory stores program instructions, and the processor is configured to perform the method for matching medical data according to any one of the embodiments of the present disclosure by loading and executing the program instructions in the memory. Of course, as is known to those skilled in the art, the computer system may also include other components, such as various input output components, communication components, and the like. Since these components can be components in the existing computer system, they are not repeated herein.

It is to be understood that the foregoing embodiments of the present disclosure are merely exemplary embodiments employed to describe the principles of the present disclosure. However, the present disclosure is not limited thereto. For those of ordinary skill in the art, various modifications and improvements may be made without departing from the spirit and essence of the present disclosure, and these modifications and improvements are also deemed to be within the scope of protection of the present disclosure. The protection scope of the present disclosure is limited only by the meaning of the language expression of the appended claims and their equivalents.

**Claims**

1. A device for matching medical data comprising:

a computing device configured to compute an overall similarity between a current object and each object in a set of objects, wherein the overall similarity is determined by one or more of an original region similarity, a living region similarity, a relevant physiological parameter similarity and a critical time similarity between the current object and each object in the set of objects; and

a selector configured to select, from the set of objects, an object with the overall similarity greater than a first threshold or a predetermined number of or a predetermined proportion of objects with the overall similarities being ranked on the top, to form a reference object subset for the current object.

2. The device according to claim 1, wherein the overall similarity is computed as a weighted sum of one or more of the original region similarity, the living region similarity, the relevant physiological parameter similarity and the critical time similarity.

3. The device according to claim 1, wherein the original region similarity represents proximity between geographical locations of ancestral homes of the objects.

4. The device according to claim 3, wherein the original region similarity is computed based on following formula:

$$OH_{i,j} = \left| \frac{L - OD_{i,j}}{L + OD_{i,j}} \right|,$$

wherein $OH_{i,j}$ represents the original region similarity between the objects i and j, $OD_{i,j}$ represents a distance between the ancestral homes of the objects i and j, and L represents a half of the earth's equatorial circumference.

5. The device according to claim 1, wherein the living region similarity represents proximity between geographical locations of living regions of the objects.

6. The device according to claim 5, wherein the living region similarity is computed based on following formula:

$$LA_{i,j} = \left| \frac{L - LD_{i,j}}{L + LD_{i,j}} \right|,$$

wherein $LA_{i,j}$ represents the living region similarity between the objects i and j, $LD_{i,j}$ represents a distance between the living regions of the objects i and j, and L represents a half of the earth's equatorial circumference.

7. The device according to claim 1, wherein the relevant physiological parameter similarity represents a similarity degree between relevant physiological parameters of the objects.

8. The device according to claim 7, wherein the relevant physiological parameter similarity is computed based on following formula:

$$ST_{i,j} = \frac{2S_i \cdot S_j^T}{(S_i \cdot C^T + S_j \cdot C^T)},$$

wherein $ST_{i,j}$ represents the relevant physiological parameter similarity between the objects i and j, $S_i$ represents a relevant physiological parameter vector of the object i, $S_j^T$ represents a transposition of the relevant physiological parameter vector of the object j, $C^T$ represents a transposition of a vector with all elements being 1, and wherein each element of the relevant physiological parameter vector corresponds to one symptom, and if an object has a certain symptom, the element corresponding to the symptom of the relevant physiological parameter vector of the object is set to 1, otherwise the element is set to 0.

9. The device according to claim 1, wherein the critical time similarity represents a similarity degree between the durations for disease symptoms of the objects.

10. The device according to claim 9, wherein the critical time similarity is computed based on following formula:

$$TF_{i,j} = \sum_{k=1}^{n}(1 - \frac{|T_{ik}-T_{jk}|}{T_{ik}+T_{jk}})/n,$$

wherein $TF_{i,j}$ represents the critical time similarity between the objects i and j, $T_{ik}$ represents a duration for the $k^{th}$ symptom of the object i, $T_{jk}$ represents a duration for the $k^{th}$ symptom of the object j, and n represents a total number of symptoms.

11. A method for matching medical data, comprising:

computing an overall similarity between a current object and each object in a set of objects, wherein the overall similarity is determined by one or more of an original region similarity, a living region similarity, a relevant physiological parameter similarity, and a critical time similarity; and
selecting, from the set of objects, an object with the overall similarity greater than a first threshold or a predetermined number of or a predetermined proportion of objects with the overall similarities being ranked on the top, to form a reference object subset for the current object.

12. The method according to claim 11, wherein the overall similarity is computed as a weighted sum of one or more of the original region similarity, the living region similarity, the relevant physiological parameter similarity, and the critical time similarity.

13. The method according to claim 11, wherein the original region similarity represents proximity between geographical locations of ancestral homes of the objects.

14. The method according to claim 13, wherein the original region similarity is computed based on following formula:

$$OH_{i,j} = \left|\frac{L-OD_{i,j}}{L+OD_{i,j}}\right|,$$

wherein $OH_{i,j}$ represents the original region similarity between the objects i and j, $OD_{i,j}$ represents a distance between the ancestral homes of the objects i and j, and L represents a half of the earth's equatorial circumference.

15. The method according to claim 11, wherein the living region similarity represents proximity between geographical locations of living regions of the objects.

16. The method according to claim 15, wherein the living region similarity is computed based on following formula:

$$LA_{i,j} = \left|\frac{L-LD_{i,j}}{L+LD_{i,j}}\right|,$$

wherein $LA_{i,j}$ represents the living region similarity between the objects i and j, $LD_{i,j}$ represents a distance between the living regions of the objects i and j, and L represents a half of the earth's equatorial circumference.

17. The method according to claim 11, wherein the relevant physiological parameter similarity represents a similarity degree between disease symptoms of the objects.

18. The method according to claim 17, wherein the relevant physiological parameter similarity is computed based on following formula:

$$ST_{i,j} = \frac{2S_i \cdot S_j^T}{(S_i \cdot C^T + S_j \cdot C^T)},$$

wherein $ST_{i,j}$ represents the relevant physiological parameter similarity between the objects i and j, $S_i$ represents a relevant physiological parameter vector of the object i, $S_j^T$ represents a transposition of the relevant physiological parameter vector of the object j, $C^T$ represents a transposition of a vector with all elements being 1, and wherein each element of the relevant physiological parameter vector corresponds to one symptom, and if an object has a certain symptom, the element corresponding to the symptom of the relevant physiological parameter vector of the object is set to 1, otherwise the element is set to 0.

19. The method according to claim 11, wherein the critical time similarity represents a similarity degree between the durations for disease symptoms of the objects.

20. The method according to claim 19, wherein the critical time similarity is computed based on following formula:

$$TF_{i,j} = \sum_{k-1}^{n}(1 - \frac{|T_{ik}-T_{jk}|}{T_{ik}+T_{jk}})/n,$$

wherein $TF_{i,j}$ represents the critical time similarity between the objects i and j, $T_{ik}$ represents a duration for the $k^{th}$ symptom of the object i, $T_{jk}$ represents a duration for the $k^{th}$ symptom of the object j, and n represents a total number of symptoms.

21. A computer system, comprising a processor and a memory connected to the processor, wherein the memory stores program instructions, and the processor is configured to perform the method according to any one of claims 11-20 by loading and executing the program instructions in the memory.

22. A storage medium, storing program instructions, wherein the program instructions are loaded and executed by a processor to perform the method according to any one of claims 11-20.

EP 3 644 199 A1

100

Device for matching medical data

110

Data of the current object

Overall similarity

120

Reference object subset

Computing Device

Selector

Data of the set of objects

130

Object Database

FIG. 1

COMPUTE AN OVERALL SIMILARITY BETWEEN A CURRENT OBJECT AND EACH OBJECT IN A SET OF OBJECTS, WHEREIN THE OVERALL SIMILARITY IS DETERMINED BY ONE OR MORE OF AN ORIGINAL REGION SIMILARITY, A LIVING REGION SIMILARITY, A RELEVANT PHYSIOLOGICAL PARAMETER SIMILARITY, AND A CRITICAL TIME SIMILARITY

210

SELECT, FROM THE SET OF OBJECTS, AN OBJECT WITH THE OVERALL SIMILARITY GREATER THAN A FIRST THRESHOLD OR A PREDETERMINED NUMBER OF OR A PREDETERMINED PROPORTION OF OBJECTS WITH THE OVERALL SIMILARITIES BEING RANKED ON THE TOP, TO FORM A REFERENCE OBJECT SUBSET FOR THE CURRENT OBJECT

220

FIG. 2

<div align="center">

# INTERNATIONAL SEARCH REPORT

</div>

| International application No. |
| --- |
| PCT/CN2018/074158 |

### A.   CLASSIFICATION OF SUBJECT MATTER

G06F 19/00 (2018.01) i

According to International Patent Classification (IPC) or to both national classification and IPC

### B.   FIELDS SEARCHED

Minimum documentation searched (classification system followed by classification symbols)

G06F

Documentation searched other than minimum documentation to the extent that such documents are included in the fields searched

Electronic data base consulted during the international search (name of data base and, where practicable, search terms used)

CNTXT, CNABS, DWPI, SIPOABS, CNKI, VEN, PATENTICS: 病例, 相似, 阈值, 距离, case, comparability, threshold, distance, interval

### C.   DOCUMENTS CONSIDERED TO BE RELEVANT

| Category* | Citation of document, with indication, where appropriate, of the relevant passages | Relevant to claim No. |
| --- | --- | --- |
| X | CN 104866979 A (SU, Rui), 26 August 2015 (26.08.2015), claims 1-7, and description, paragraph 19 | 1-22 |
| A | CN 105608327 A (HUASHAN HOSPITAL AFFILIATED TO FUDAN UNIVERSITY), 25 May 2016 (25.05.2016), entire document | 1-22 |
| A | CN 103761344 A (SHENZHEN AIKANG INFORMATION TECHNOLOGY CO., LTD.), 30 April 2014 (30.04.2014), entire document | 1-22 |

☐ Further documents are listed in the continuation of Box C.        ☒  See patent family annex.

| | |
| --- | --- |
| *    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| 10 April 2018 | 26 April 2018 |

| Name and mailing address of the ISA | Authorized officer |
| --- | --- |
| State Intellectual Property Office of the P. R. China No. 6, Xitucheng Road, Jimenqiao Haidian District, Beijing 100088, China Facsimile No. (86-10) 62019451 | ZHANG, Guihua Telephone No. (86-10) 62089416 |

Form PCT/ISA/210 (second sheet) (January 2015)

## INTERNATIONAL SEARCH REPORT
### Information on patent family members

| | | International application No. |
|---|---|---|
| | | PCT/CN2018/074158 |

| Patent Documents referred in the Report | Publication Date | Patent Family | Publication Date |
|---|---|---|---|
| CN 104866979 A | 26 August 2015 | None | |
| CN 105608327 A | 25 May 2016 | None | |
| CN 103761344 A | 30 April 2014 | None | |

Form PCT/ISA/210 (patent family annex) (January 2015)